(19) Europäisches Patentamt
European Patent Office
Office européen des brevets

(11) Veröffentlichungsnummer: **0 030 277**
**B1**

# (12) EUROPÄISCHE PATENTSCHRIFT

(45) Veröffentlichungstag der Patentschrift: **27.07.83**

(51) Int. Cl.³: **C 07 C 13/28,** C 09 K 3/34, G 02 F 1/13

(21) Anmeldenummer: **80106847.9**

(22) Anmeldetag: **06.11.80**

## (54) Partiell hydrierte Oligo-1,4-phenylene, diese enthaltende Dielektrika und elektrooptisches Anzeigeelement.

(30) Priorität: **05.12.79 DE 2948836**

(43) Veröffentlichungstag der Anmeldung: **17.06.81 Patentblatt 81/24**

(45) Bekanntmachung des Hinweises auf die Patenterteilung: **27.07.83 Patentblatt 83/30**

(84) Benannte Vertragsstaaten: **AT CH DE FR GB IT LI NL**

(56) Entgegenhaltungen:
**DD-A-134 114**
**DE-A-2 356 085**

**Die Akte enthält technische Angaben, die nach dem Eingang der Anmeldung eingereicht wurden und die nicht in dieser Patentschrift enthalten sind.**

(73) Patentinhaber: **Merck Patent Gesellschaft mit beschränkter Haftung, Frankfurter Strasse 250, D-6100 Darmstadt (DE)**

(72) Erfinder: **Eidenschink, Rudolf, Dr., Dessauer Strasse 57, D-6110 Dieburg (DE)**
Erfinder: **Pohl, Ludwig, Dr., Niebergallweg 5, D-6100 Darmstadt (DE)**
Erfinder: **Römer, Michael, Dr., Rathenaustrasse 52, D-6054 Rodgau 1 (DE)**
Erfinder: **del Pino, Fernando, Diaz de Madrid 1166, Quito (EC)**

**EP 0 030 277 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Partiell hydrierte Oligo-1,4-phenylene, diese enthaltende Dielektrika und elektrooptisches Anzeigeelement

Für elektrooptische Anzeigeelemente werden in zunehmendem Maße die Eigenschaften nematischer oder nematisch-cholesterischer flüssigkristalliner Materialien ausgenutzt, ihre optischen Eigenschaften wie Lichtabsorption, Lichtstreuung, Doppelbrechung, Reflexionsvermögen oder Farbe unter dem Einfluß elektrischer Felder signifikant zu verändern. Die Funktion derartiger Anzeigeelemente beruht dabei beispielsweise auf den Phänomen der dynamischen Streuung, der Deformation aufgerichteter Phasen, dem Schadt-Helfrich-Effekt in der verdrillten Zelle oder dem cholesterisch-nematischen Phasenübergang.

Für die technische Anwendung dieser Effekt in elektronischen Bauelementen werden flüssigkristalline Dielektrika benötigt, die einer Vielzahl von Anforderungen genügen müssen. Besonders wichtig ist hier die chemische Beständigkeit gegenüber Feuchtigkeit, Luft und physikalischen Einflüssen wie Wärme, Strahlung um infraroten, sichtbaren und ultravioletten Bereich und elektrische Gleich- und Wechselfelder. Ferner wird von technisch verwendbaren flüssigkristallinen Dielektrika eine flüssigkristalline Mesophase im Temperaturbereich von mindestens +10°C bis +50°C, bevorzugt von 0°C bis 60°C, und eine möglichst niedrige Viskosität bei Raumtemperatur, die vorzugsweise nicht mehr als $70 \cdot 10^{-3}$ Pa · s betragen soll, gefordert. Schließlich dürfen sie im Bereich des sichtbaren Lichtes keine Eigenabsorption aufweisen, d. h. sie müssen farblos sein.

Es ist bereits eine Anzahl von flüssigkristallinen Verbindungen bekannt, die den an Dielektrika für elektronische Bauelemente gestellten Stabilitätsanforderungen genügen und auch farblos sind. Hierzu gehören insbesondere die in der DE-OS 2 139 628 beschriebenen p,p'-disubstituierten Benzoesäurephenylester und die in der DE-OS 2 636 684 beschriebenen p,p'-disubstituierten Phenylcyclohexanderivate. In beiden genannten Verbindungsklassen wie auch in anderen bekannten Reihen von Verbindungen mit flüssigkristalliner Mesophase gibt es keine Einzelverbindungen, die in dem geforderten Temperaturbereich von 10°C bis 60°C eine flüssigkristalline nematische Mesophase ausbilden. Es werden daher in der Regel Mischungen von zwei oder mehreren Verbindungen hergestellt, um als flüssigkristalline Dielektrika verwendbare Substanzen zu erhalten. Hierzu mischt man gewöhnlich mindestens eine Verbindung mit niedrigem Schmelz- und Klarpunkt mit einer anderen mit deutlich höherem Schmelz- und Klärpunkt.

Hierbei wird normalerweise ein Gemisch erhalten, dessen Schmelzpunkt unter dem der niedriger schmelzenden Komponente liegt, während der Klärpunkt zwischen den Klärpunkten der Komponenten liegt. Optimale Dielektrika lassen sich jedoch auf diese Weise nicht leicht herstellen, da die Komponenten mit den hohen Schmelz- und Klärpunkten den Gemischen häufig auch eine hohe Viskosität verleihen. Dadurch werden die Schaltzeiten der damit hergestellten elektrooptischen Anzeigeelemente in unerwünschter Weise verlängert.

Der Erfindung liegt die Aufgabe zugrunde, flüssigkristalline Dielektrika herzustellen, die eine nematische Phase in geforderten Temperaturbereichen aufweisen und in Flüssigkristallzellen bei Raumtemperatur ausreichend kurze Schaltzeiten ermöglichen. Weiterhin ist es für alle eingangs genannten Arten von Anzeigeelementen von Bedeutung, daß die Kontrast-Spannungskurve im Bereich der Schwellenspannung möglichst steil ist, d. h., daß ein geringfügiges Überschreiten der Schwellenspannung sofort die Anzeige zu möglichst vollem Kontrast aktiviert; ferner soll die Schwellenspannung selbst nur eine möglichst geringe Temperaturabhängigkeit besitzen, damit zur Aktivierung der Anzeige insbesondere bei tiefen Temperaturen nicht wesentlich höhere Schwellenspannungen nötig sind als zum Beispiel bei Raumtemperatur.

Es wurde nun gefunden, daß die partiell hydrierten Oligo-1,4-phenylene der Formel (I),

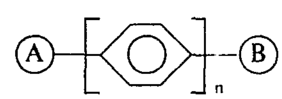 (I)

worin n 1 oder 2 ist und die Ringe A und B, die gleich oder verschieden sind, trans-4-Alkylcyclohexyl oder 4-Alkylcyclohexen-1-yl bedeuten, wobei die Alkylgruppen jeweils bis zu 10 Kohlenstoffatome enthalten, hervorragend als Mischungskomponenten zur Herstellung flüssigkristalliner Dielektrika mit bisher unerreicht geringer Abhängigkeit der Schwellenspannung von der Temperatur geeignet sind. Darüber hinaus weisen insbesondere die Verbindungen der Formel (I), in denen n = 2 ist, sehr hohe Klärpunkte auf, zumeist deutlich über 200°C; sie können daher vorteilhaft zur Erweiterung des Temperaturbereichs der nematischen Phase von flüssigkristallinen Dielektrika verwendet werden.

Gegenstand der Erfindung sind somit die partiell hydrierten Oligo-1,4-phenyle der Formel (I) und ihre Verwendung als Komponenten flüssigkristalliner Dielektrika. Gegenstand der Erfindung sind weiterhin flüssigkristalline Dielektrika mit einem Gehalt an mindestens einem partiell hydrierten Oligo-1,4-phenylen der Formel (I) sowie elektrooptische Anzeigeelemente auf der Basis einer Flüssigkristallzelle, die ein derartiges flüssigkristallines Dielektrikum enthalten.

Die erfindungsgemäßen partiell hydrierten Oligo-1,4-phenylene umfassen somit 1,4-Bis-(trans-4-Alkylcyclohexyl)-benzole der Formel '(Ia)

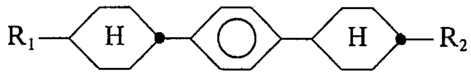

(Ia)

worin $R_1$ und $R_2$ gleich oder verschieden sind und Alkylgruppen mit bis zu 10 Kohlenstoffatomen bedeuten,

1,4-Bis-(4-Alkylcyclohexen-1-yl)-benzole der Formel (Ib),

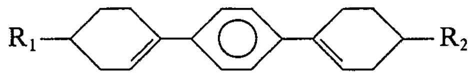

(Ib)

4,4'-Bis-(trans-4-Alkylcyclohexyl)-biphenyle der Formel (Ic),

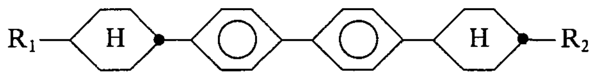

(Ic)

4,4'-Bis-(4-Alkylcyclohexen-1-yl)-biphenyle der Formel (Id),

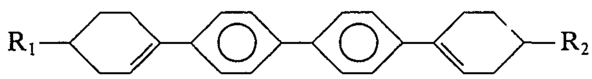

(Id)

1-(trans-4-Alkylcyclohexyl)-4-(4-alkylcyclohexen-1-yl)-benzole der Formel (Ie),

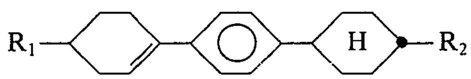

(Ie)

und 4-(trans-4-Alkylcyclohexyl)-4'-(4-alkylcyclohexen-1-yl)-biphenyle der Formel (If),

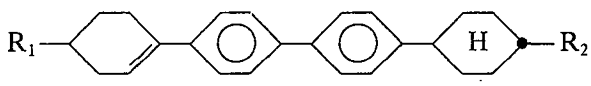

(If)

wobei $R_1$ und $R_2$ in den Formeln (Ib) bis (If) die bei der Formel (Ia) angegebene Bedeutung besitzen.

Soweit die erfindungsgemäßen Verbindungen Cyclohexanringe enthalten, sind diese trans-1,4-ständig disubstituiert; dies ist in den Formelbildern durch den schwarzen Punkt an der rechten Seite der Cyclohexanringe zum Ausdruck gebracht. Die Verbindungen der Formeln (Ia), (Ib) und (Ie) haben in der Regel Schmelz- und Klärpunkte bei tieferen Temperaturen als die analog substituierten Biphenylderivate der Formel (Ic), (Id) und (If).

Sie besitzen jedoch gleichzeitig auch eine wesentlich geringere Viskosität und werden daher bevorzugt als viskositätserniedrigende Komponenten flüssigkristalliner Dielektrika verwendet, deren übrige Bestandteile hohe Klärpunkte aufweisen. Demgegenüber haben die Biphenylderivate (Ic), (Id) und (If) einen extrem breiten Mesophasenbereich, der in der Regel 150 Grad und mehr umfaßt. Sie werden daher bevorzugt zur Erweiterung des Mesophasenbereichs flüssigkristalliner Dielektrika eingesetzt, wobei sie die Viskosität deutlich weniger erhöhen als andere bekannte flüssigkristalline Verbindungen mit hohem Klärpunkt, die bisher für diesen Zweck eingesetzt wurden. Dabei wurde überraschenderweise gefunden, daß erfindungsgemäße Dielektrika mit einer oder mehreren Verbindungen der Formeln (I) eine besonders steile Kontrast-Spannungs-Kurve und eine sehr geringe Temperaturabhängigkeit der Schwellenspannung zeigen.

Die Alkylgruppen in den Verbindungen der Formel (I) können geradkettig oder verzweigt sein, wobei die Verbindungen mit zwei geradkettigen Flügelgruppen bevorzugt sind. Verbindungen der Formel (I), in denen einer der Reste $R_1$ und $R_2$ verzweigt ist, können gelegentlich als chirale Dotierstoffe verwendet werden, wenn sie durch die Kettenverzweigung optisch aktiv sind. In keinem Fall enthält eine verzweigte Flügelgruppe mehr als eine Kettenverzweigung; bevorzugte verzweigte Flügelgruppen sind solche, in denen sich eine Methyl- oder Ethylgruppe in 1- oder 2-Stellung einer längeren Kohlenstoffkette befindet, beispielsweise 1-Methylpropyl, 2-Methylpropyl, 2-Methylbutyl, 2-Methylpentyl, 2-Ethylhexyl, 1-Methylheptyl oder 2-Methylheptyl. In den erfindungsgemäßen Verbindungen enthalten die Flügelgruppen $R_1$ und $R_2$ jeweils bis zu 10 Kohlenstoffatome, zusammen also 2–20 Kohlenstoffatome. Bevorzugte Verbindungen der Formel (I) sind jedoch die, in denen $R_1$ und $R_2$ zusammen 3–16 Kohlenstoffatome, insbesondere 4–13 Kohlenstoffatome enthalten.

Die erfindungsgemäßen Verbindungen werden in für derartige Substanzen üblicher Weise hergestellt.

So werden die Verbindungen der Formel (Ia) erhalten, indem ein trans-(4-Alkylcyclohexyl)-benzol der Formel (II)

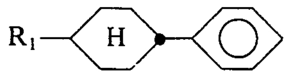

(II)

mit Brom in Tetrachlorkohlenstoff in Gegenwart von Eisenpulver zum 4-(trans-4-Alkylcyclohexyl)-brombenzol umgesetzt wird und dieses in einer Grignard-Reaktion durch Umsetzung mit Magnesium in Gegenwart eines Ethers, Zusatz eines 4-Alkylcyclohexanons, Hydrolyse, Hydrierung und gegebenenfalls cis-trans-Isomerisierung und Isomerentrennung in ein 1,4-Bis-(trans-4-Alkylcyclohexyl)-benzol (Ia) übergeführt wird. Verbindungen der Formel (Ie) werden hergestellt, indem in der vorstehenden Reaktionsfolge zur Herstellung der Verbindungen (Ia) anstelle der Hydrierung und der nachfolgenden Schritte eine Wasserabspaltung durch Behandlung mit einer Säure, zum Beispiel p-Toluolsulfonsäure vorgenommen wird. Die Verbindungen der Formel (Ib) werden analog zu denen der Formel (Ie) erhalten, wenn als Ausgangsmaterial ein 4-(4-Alkylcyclohexen-1-yl)-brombenzol der Formel (IIa) eingesetzt wird.

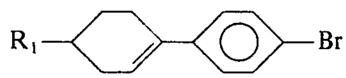

(IIa)

Die Verbindungen der Formel (IIa) sind aus der offengelegten europäischen Patentanmeldung Nr. 2136 bekannt. Zur Herstellung von solchen Verbindungen der Formel (Ib), in denen $R_1$ und $R_2$ gleich sind, kann man auch vom 1,4-Dibrombenzol ausgehen, das mit der zweifachen molaren Menge Magnesium und anschließend mit der zweifachen molaren Menge 4-Alkylcyclohexanon umgesetzt wird. Anschließend werden die Schritt der Hydrolyse und der Wasserabspaltung in der üblichen Weise durchgeführt.

Die Herstellung der Biphenylderivate (Ic), (Id) und (If) erfolgt analog zu der der Benzolderivate (Ia), (Ib) bzw. (Ie), wobei als Ausgangsmaterialen 4-(trans-4-Alkylcyclohexyl)-biphenyle der Formel (III),

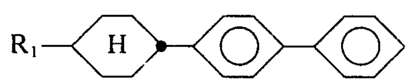

(III)

4-(4-Alkylcyclohexen-1-yl)-4'-brombiphenyle der Formel (IIIa)

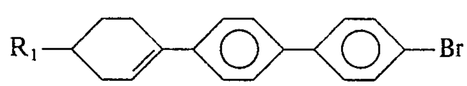

(IIIa)

beziehungsweise 4,4'-Dibrombiphenyl verwendet werden.

Die erfindungsgemäßen flüssigkristallinen Dielektrika bestehen aus zwei oder mehr Komponenten, darunter mindestens eine der Formel (I). Gegebenenfalls verwendete weitere Komponenten sind vorzugsweise nematische oder nematogene Substanzen aus den Klassen der Azobenzole, Azoxybenzole, Biphenyle, Schiffsche Basen, insbesondere Benzyliden-Derivate, Phenylbenzoate, Phenylpyrimidine, Phenylcyclohexane, gegebenenfalls halogenierte Stilbene, Diphenylacetylen-Derivate, Diphenylnitrone und substituierten Zimtsäuren. Die wichtigsten als derartige weitere Komponenten in Frage kommenden Verbindungen lassen sich durch die Formel (IV) charakterisieren:

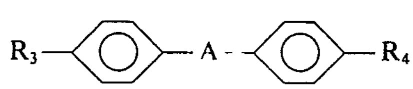

(IV)

worin

A $-CH{=}CH-$ 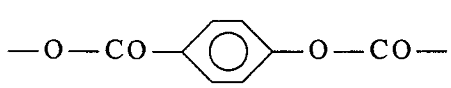

$-CX{=}CH-$ 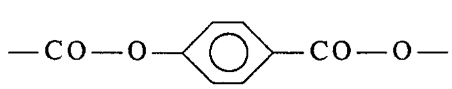

$-CH{=}CX-$ 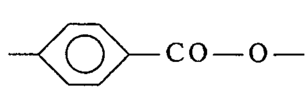

$-C{\equiv}C-$ 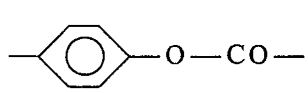

—N=N—

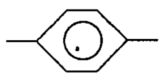 —CO—S—

—N(O)=N—

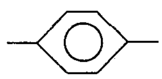 —S—CO—

—N=N(O)—

—CH=N—

—O—CO—

—N=CH—

—CO—O—

—CH=N(O)—

—S—CO—

—N(O)=CH—

—CO—S—

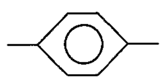 oder eine C–C-Einfachverbindung

bedeutet. Weitere mögliche Komponenten der erfindungsgemäßen Dielektrika sind solche Verbindungen der Formel (IV), in denen einer oder mehrere Phenylringe durch eine entsprechende Zahl von trans-Cyclohexylringen ersetzt sind; ferner kann auch einer dieser Ringe ein 2,5-disubstituierter Pyrimidinring sein.

X bedeutet Halogen, vorzugsweise Cl, oder –CN. $R_3$ und $R_4$ sind gleich oder verschieden und können Alkyl-, Alkoxy-, Alkanoyl-, Alkanoyloxy- oder Alkoxycarbonyloxyreste mit bis zu 18, vorzugsweise bis zu 8 C-Atomen bedeuten; weiterhin kann auch einer dieser Reste –CN, –NC, $NO_2$, $CF_3$ oder Halogen bedeuten.

Bei den meisten dieser Verbindungen sind $R_3$ und $R_4$ vorzugsweise verschieden, wobei einer der Reste meist eine Alkyl- oder Alkoxygruppe bedeutet. Aber auch eine große Zahl anderer Varianten der vorgesehenen Substituenten sind gebräuchlich. Viele solcher Substanzen sind im Handel erhältlich.

Die erfindungsgemäßen Dielektrika enthalten in der Regel mindestens 30, vorzugsweise 50–99, insbesondere 60–98 Gewichtsteile der Verbindungen der Formel (I) und (IV). Hiervon entfallen bevorzugt 3 bis 50, insbesondere 5 bis 25 Gewichtsteile auf eine oder mehrere Verbindungen der Formel (I). Es werden von der Erfindung auch solche flüssigkristallinen Dielektrika umfaßt, denen beispielsweise zu Dotierungszwecken nur weniger als 3 Gewichtsteile, zum Beispiel 0,1 bis 2 Gewichtsteile einer oder mehrerer Verbindungen der Formel (I) zugesetzt worden sind. Andererseits können die Verbindungen der Formel (I) bis zu 80 Gewichtsprozent der erfindungsgemäßen Dielektrika ausmachen.

Die Herstellung der erfindungsgemäßen Dielektrika erfolgt in an sich üblicher Weise. In der Regel wird die gewünschte Menge der in geringerer Menge verwendeten Komponenten in der den Hauptbestandteil ausmachenden Komponente gelöst, zweckmäßig bei erhöhter Temperatur. Wenn dabei eine Temperatur oberhalb des Klärpunkts des Hauptbestandteils gewählt wird, kann die Vollständigkeit des Lösevorgangs besonders leicht beobachtet werden.

Es ist jedoch auch möglich, Lösungen der Komponenten der Formel (I) und (IV) in einem geeigneten organischen Lösungsmittel, zum Beispiel Aceton, Chloroform oder Methanol, zu mischen und das Lösungsmittel nach gründlicher Durchmischung wieder zu entfernen, beispielsweise durch Destillation unter vermindertem Druck. Selbstverständlich muß bei dieser Verfahrensweise darauf geachtet werden, daß durch das Lösungsmittel keine Verunreinigungen oder unerwünschten Dotierungsstoffe eingeschleppt werden.

Durch geeignete Zusätze können die flüssigkristallinen Dielektrika nach der Erfindung so modifiziert werden, daß sie in allen bisher bekanntgewordenen Arten von Flüssigkristallanzeigeelementen verwendet werden können. Derartige Zusätze sind dem Fachmann bekannt und sind in der einschlägigen Literatur ausführlich beschrieben. Beispielsweise können Substanzen zur Veränderung der dielektrischen Anisotropie, der Viskosität, der Leitfähigkeit und/oder der Orientierung der nematischen Phasen zugesetzt werden. Derartige Substanzen sind zum Beispiel in der DE-OS 2 209 127, 2 240 864, 2 321 632, 2 338 281 und 2 450 088 beschrieben.

Die folgenden Beispiele sollen die Erfindung erläutern, ohne sie zu begrenzen. In den Beispielen bedeuten F. den Schmelzpunkt und K. den Klärpunkt einer flüssigkristallinen Substanz in Grad Celsius; Siedetemperaturen sind mit Kp. bezeichnet. Wenn nichts anderes angegeben ist, bedeuten Angaben von Teilen oder Prozent Gewichtsteile bzw. Gewichtsprozent.

## Beispiel 1

a) Zu einer Lösung von 230 g (trans-4-n-Pentylcyclohexyl)-benzol in 750 ml Tetrachlorkohlenstoff werden in Gegenwart von 15 g Eisenspänen unter Rühren 160 g Brom so zugetropft, daß die Temperatur 30° nicht übersteigt. Anschließend wird das Reaktionsgemisch noch 10 Minuten zum Sieden erhitzt, dann filtriert und fraktioniert destilliert. Nach dem Abdestillieren des Lösungsmittels

werden unter vermindertem Druck 163 g 4-(trans-4-n-Pentylcyclohexyl)-brombenzol abdestilliert; Kp. 130–135°/0,1 Torr. ·

b) Zu einer aus 31 g 4-(trans-4-n-Pentylcyclohexyl)-brombenzol und 3 g Magnesiumspänen in 100 ml Tetrahydrofuran bereiten Grignard-Lösung wird bei Raumtemperatur unter Rühren eine Lösung von 18 g 4-n-Pentylcyclohexanon in 30 ml Tetrahydrofuran getropft. Anschließend wird mit einer 10% wäßrigen Lösung von Ammoniumchlorid hydrolysiert, die organische Phase abgetrennt, diese zweimal mit je 50 ml Wasser gewaschen, über Natriumsulfat getrocknet und eingedampft. Der Rückstand wird in 300 ml Ethanol gelöst und die Lösung in Gegenwart von 4 g Palladiumkohle (5% Pd) 48 Stunden bei Raumtemperatur hydriert. Anschließend wird vom Katalysator abfiltriert, das Lösungsmittel abdestilliert, der Rückstand in 50 ml Dimethylsulfoxid aufgenommen und unter Feuchtigkeitsausschluß in Gegenwart von 7 g Kalium-tert.butylat 16 Stunden auf 100° erwärmt. Nach dem Abkühlen wird das Reaktionsgemisch in 300 ml Eiswasser gegossen und die wäßrige Phase zweimal mit je 100 ml Dichlormethan extrahiert. Die Extrakte werden mit 100 ml Wasser gewaschen, über Calciumchlorid getrocknet und eingedampft. Das zurückbleibende 1,4-Bis-(trans-4-n-Pentylcyclohexyl)-benzol wird aus Ethanol umkristallisiert; Ausbeute 19 g, F. 50°, K. 196°.

Analog werden hergestellt:

1,4-Bis-(4-trans-Methylcyclohexyl)-benzol,
1-(trans-4-Ethylcyclohexyl)-4-(trans-4-methylcyclohexyl)-benzol,
1-(trans-4-n-Propylcyclohexyl)-4-(trans-4-methylcyclohexyl)-benzol,
1-(trans-4-n-Butylcyclohexyl)-4-(trans-4-methylcyclohexyl)-benzol,
1-(trans-4-n-Pentylcyclohexyl)-4-(trans-4-methylcyclohexyl)-benzol,
1-(trans-4-n-Hexylcyclohexyl)-4-(trans-4-methylcyclohexyl)-benzol,
1-(trans-4-n-Heptylcyclohexyl)-4-(trans-4-methylcyclohexyl)-benzol,
1-(trans-4-n-Octylcyclohexyl)-4-(trans-4-methylcyclohexyl)-benzol,
1-[trans-4-(2-Methylbutyl)-cyclohexyl]-4-(trans-4-methylcyclohexyl)-benzol,

1,4-Bis-(trans-4-Ethylcyclohexyl)-benzol,
1-(trans-4-n-Propylcyclohexyl)-4-(trans-4-ethylcyclohexyl)-benzol,
1-(trans-4-n-Butylcyclohexyl)-4-(trans-4-ethylcyclohexyl)-benzol,
1-(trans-4-n-Pentylcyclohexyl)-4-(trans-4-ethylcyclohexyl)-benzol,
1-(trans-4-n-Hexylcyclohexyl)-4-(trans-4-ethylcyclohexyl)-benzol,
1-(trans-4-n-Heptylcyclohexyl)-4-(trans-4-ethylcyclohexyl)-benzol,
1-(trans-4-n-Octylcyclohexyl)-4-(trans-4-ethylcyclohexyl)-benzol,
1-[trans-4-(2-Methylbutyl)-cyclohexyl]-4-(trans-4-ethylcyclohexyl)-benzol,
1-[trans-4-(2-Ethylhexyl)-cyclohexyl]-4-(trans-4-ethylcyclohexyl)-benzol,

1,4-Bis-(trans-4-n-Propylcyclohexyl)-benzol, F. 77°, K. 194°;
1-(trans-4-n-Butylcyclohexyl)-4-(trans-4-n-propylcyclohexyl)-benzol,
1-(trans-4-n-Pentylcyclohexyl)-4-(trans-4-n-propylcyclohexyl)-benzol,
1-(trans-4-n-Hexylcyclohexyl)-4-(trans-4-n-propylcyclohexyl)-benzol,
1-(trans-4-n-Heptylcyclohexyl)-4-(trans-4-n-propylcyclohexyl)-benzol,
1-(trans-4-n-Octylcyclohexyl)-4-(trans-4-n-propylcyclohexyl)-benzol,
1-[trans-4-(2-Methylbutyl)-cyclohexyl]-4-n-(trans-4-n-propyl-cyclohexyl)-benzol,
1-[trans-4-(2-Ethylhexyl)-cyclohexyl]-4-(trans-4-n-propyl-cyclohexyl)-benzol,

1,4-Bis-(trans-4-n-Butylcyclohexyl)-benzol,
1-(trans-4-n-Pentylcyclohexyl)-4-(trans-4-n-butylcyclohexyl)-benzol,
1-(trans-4-n-Hexylcyclohexyl)-4-(trans-4-n-butylcyclohexyl)-benzol,
1-(trans-4-n-Heptylcyclohexyl)-4-(trans-4-n-butylcyclohexyl)-benzol,
1-(trans-4-n-Octylcyclohexyl)-4-(trans-4-n-butylcyclohexyl)-benzol,
1-[trans-4-(2-Methylbutyl)-cyclohexyl]-4-(trans-4-n-butylcyclohexyl)-benzol,
1-[trans-4-(2-Ethylhexyl)-cyclohexyl]-4-(trans-4-n-butylcyclohexyl)-benzol,

1-(trans-4-n-Hexylcyclohexyl)-4-(trans-4-n-pentylcyclohexyl)-benzol,
1-(trans-4-n-Heptylcyclohexyl)-4-(trans-4-n-pentylcyclohexyl)-benzol,
1-(trans-4-n-Octylcyclohexyl)-4-(trans-4-n-pentylcyclohexyl)-benzol,
1-[trans-4-(2-Methylbutyl)-cyclohexyl]-4-(trans-4-n-pentylcyclohexyl)-benzol,
1-(trans-4-(2-Ethylhexyl)-cyclohexyl]-4-(trans-4-n-pentylcyclohexyl)-benzol,

1,4-Bis-(trans-4-n-Hexylcyclohexyl)-benzol,
1-(trans-4-n-Heptylcyclohexyl)-4-(trans-4-n-hexylcyclohexyl)-benzol,
1-(trans-4-n-Octylcyclohexyl)-4-(trans-4-n-hexylcyclohexyl)-benzol,

1-[trans-4-(2-Methylbutyl)-cyclohexyl]-4-trans-4-n-hexyl-cyclohexyl)-benzol,
1-[trans-4-(2-Ethylhexyl)-cyclohexyl]-4-(trans-4-n-hexylcyclohexyl)-benzol,

1,4-Bis-(trans-4-n-Heptylcyclohexyl)-benzol,
1-[trans-4-(2-Methylbutyl)-cyclohexyl]-4-(trans-4-n-heptylcyclohexyl)-benzol,

1-[trans-4-(2-Methylbutyl)-cyclohexyl]-4-(trans-4-n-octylcyclohexyl)-benzol.

Beispiel 2

a) Analog Beispiel 1(a) wird 4-(trans-4-n-Pentylcyclohexyl)-biphenyl bromiert; nach dem Abdestillieren des Tetrachlorkohlenstoffs wird jedoch das als kristalline Masse zurückbleibende 4-(trans-4-n-Pentylcyclohexyl)-4'-brombiphenyl aus Ethanol umkristallisiert; F. 148°, K. 185°.

b) Analog Beispiel 1(b) wird eine aus 38,5 g 4-(trans-4-n-Pentylcyclohexyl)-4'-brombiphenyl und 3 g Magnesium in 200 ml Tetrahydrofuran bereitete Grignard-Lösung mit 4-Methylcyclohexanon umgesetzt. Nach Hydrolyse, Hydrierung und Isomerisierung in der in Beispiel 1(b) beschriebenen Weise wird nach Umkristallisation aus Ethanol 4-(trans-4-n-Pentylcyclohexyl)-4'-(trans-4-methylcyclohexyl)-biphenyl erhalten; F. 96°, K. 284°.

Analog werden hergestellt:

4,4'-Bis-(trans-4-Methylcyclohexyl)-biphenyl,
4-(trans-4-Ethylcyclohexyl)-4'-(trans-4-methylcyclohexyl)-biphenyl,
4-(trans-4-n-Propylcyclohexyl)-4'-(trans-4-methylcyclohexyl)-biphenyl,
4-(trans-4-n-Butylcyclohexyl)-4'-(trans-4-methylcyclohexyl)-biphenyl,
4-(trans-4-n-Hexylcyclohexyl)-4'-(trans-4-methylcyclohexyl)-biphenyl,
4-(trans-4-n-Heptylcyclohexyl)-4'-(trans-4-methylcyclohexyl)-biphenyl,
4-(trans-4-n-Octylcyclohexyl)-4'-(trans-4-methylcyclohexyl)-biphenyl,
4-[trans-4-(2-Methylbutyl)-cyclohexyl]-4'-(trans-4-methylcyclohexyl)-biphenyl,
4-[trans-4-(2-Ethylhexyl)-cyclohexyl]-4'-(trans-4-methylcyclohexyl)-biphenyl,

4,4'-Bis-(trans-4-Ethylcyclohexyl)-biphenyl,
4-(trans-4-n-Propylcyclohexyl)-4'-(trans-4-ethylcyclohexyl)-biphenyl,
4-(trans-4-n-Butylcyclohexyl)-4'-(trans-4-ethylcyclohexyl)-biphenyl,
4-(trans-4-n-Pentylcyclohexyl)-4'-(trans-4-ethylcyclohexyl)-biphenyl,
4-(trans-4-n-Hexylcyclohexyl)-4'-(trans-4-ethylcyclohexyl)-biphenyl,
4-(trans-4-n-Heptylcyclohexyl)-4'-(trans-4-ethylcyclohexyl)-biphenyl,
4-(trans-4-n-Octylcyclohexyl)-4'-(trans-4-ethylcyclohexyl)-biphenyl,
4-[trans-4-(2-Methylbutyl)-cyclohexyl]-4'-(trans-4-ethylcyclohexyl)-biphenyl,
4-[trans-4-(2-Ethylhexyl)-cyclohexyl]-4'-(trans-4-ethylcyclohexyl)-biphenyl,

4,4'-Bis-(trans-4-n-Propylcyclohexyl)-biphenyl, F. 63°, K. 308°;
4-(trans-4-n-Butylcyclohexyl)-4'-(trans-4-n-propylcyclohexyl)-biphenyl,
4-(trans-4-n-Pentylcyclohexyl)-4'-(trans-4-n-propylcyclohexyl)-biphenyl,
4-(trans-4-n-Hexylcyclohexyl)-4'-(trans-4-n-propylcyclohexyl)-biphenyl,
4-(trans-4-n-Heptylcyclohexyl)-4'-(trans-4-n-propylcyclohexyl)-biphenyl,
4-(trans-4-n-Octylcyclohexyl)-4'-(trans-4-n-propylcyclohexyl)-biphenyl,
4-[trans-4-(2-Methylbutyl)-cyclohexyl]-4'-(trans-4-n-propylcyclohexyl)-biphenyl,
4-[trans-4-(2-Ethylhexyl)-cyclohexyl]-4'-(trans-4-n-propylcyclohexyl)-biphenyl,

4,4'-Bis-(trans-4-n-Butylcyclohexyl)-biphenyl,
4-(trans-4-n-Pentylcyclohexyl)-4'-(trans-4-n-butylcyclohexyl)-biphenyl,
4-(trans-4-n-Hexylcyclohexyl)-4'-(trans-4-n-butylcyclohexyl)-biphenyl,
4-(trans-4-n-Heptylcyclohexyl)-4'-(trans-4-n-butylcyclohexyl)-biphenyl,
4-(trans-4-n-Octylcyclohexyl)-4'-(trans-4-n-butylcyclohexyl)-biphenyl,
4-[trans-4-(2-Methylbutyl)-cyclohexyl]-4'-(trans-4-n-butylcyclohexyl)-biphenyl,
4-[trans-4-(2-Ethylhexyl)-cyclohexyl]-4'-(trans-4-n-butylcyclohexyl)-biphenyl,

4,4'-Bis-(trans-4-n-Pentylcyclohexyl)-biphenyl, F. 43°, K. 300°;
4-(trans-4-n-Hexylcyclohexyl)-4'-(trans-4-n-pentylcyclohexyl)-biphenyl,F. 84°, K. 240°;
4-(trans-4-n-Heptylcyclohexyl)-4'-(trans-4-n-pentylcyclohexyl)-biphenyl,
4-(trans-4-n-Octylcyclohexyl)-4'-(trans-4-n-pentylcyclohexyl)-biphenyl,
4-[trans-4-(2-Methylbutyl)-cyclohexyl]-4'-(trans-4-n-pentylcyclohexyl)-biphenyl,
4-[trans-4-(2-Ethylhexyl)-cyclohexyl]-4'-(trans-4-n-pentylcyclohexyl)-biphenyl,

4,4'-Bis-(trans-4-n-Hexylcyclohexyl)-biphenyl,
4-(trans-4-n-Heptylcyclohexyl)-4'-(trans-4-n-hexylcyclohexyl)-biphenyl,
4-(trans-4-n-Octylcyclohexyl)-4'-(trans-4-n-hexylcyclohexyl)-biphenyl,
4-[trans-4-(2-Methylbutyl)-cyclohexyl]-4'-(trans-4-n-hexylcyclohexyl)-biphenyl,
4-[trans-4-(2-Ethylhexyl)-cyclohexyl]-4'-(trans-4-n-hexylcyclohexyl)-biphenyl,

4,4'-Bis-(trans-4-n-Heptylcyclohexyl)-biphenyl,
4-[trans-4-(2-Methylbutyl)-cyclohexyl]-4'-(trans-4-n-heptylcyclohexyl)-biphenyl,
4-[trans-4-(2-Methylbutyl)-cyclohexyl]-4'-(trans-4-n-octylcyclohexyl)-biphenyl.

## Beispiel 3

Eine Lösung von 24 g 1,4-Dibrombenzol in 30 ml Tetrahydrofuran wird langsam unter Rühren zu 48 g Magnesiumspänen in 50 ml Tetrahydrofuran getropft. Anschließend wird das Reaktionsgemisch 2 Stunden zum Sieden erhitzt und nach Abkühlen tropfenweise mit einer Lösung von 28 g 4-n-Propylcyclohexanon in 40 ml Tetrahydrofuran versetzt. Es wird noch 2 Stunden bei 60° nachgerührt und dann durch Zutropfen von 1 l 5% wäßriger Salzsäure hydrolysiert. Das hydrolysierte Reaktionsgemisch wird dreimal mit insgesamt 600 ml Diethylether extrahiert, die Extrakte mit Wasser gewaschen und eingedampft, zuletzt unter vermindertem Druck. Der sirupöse Rückstand wird in 90 ml Aceton aufgenommen, mit 5 g p-Toluol-sulfonsäure versetzt und 4 Stunden unter Rückfluß zum Sieden erhitzt. Danach wird das Reaktionsgemisch auf −5°C abgekühlt, das auskristallisierte 1,4-Bis-(4-n-Propylcyclohexen-1-yl)-benzol abfiltriert und aus Isopropylalkohol umkristallisiert; Ausbeute 17,6 g, F. 42°, K. 212°.

Analog werden hergestellt:

1,4-Bis-(4-Methylcyclohexen-1-yl)-benzol,
1,4-Bis-(4-Ethylcyclohexen-1-yl)-benzol,
1,4-Bis-(4-n-Butylcyclohexen-1-yl)-benzol,
1,4-Bis-(4-n-Pentylcyclohexen-1-yl)-benzol,
1,4-Bis-(4-n-Hexylcyclohexen-1-yl)-benzol,
1,4-Bis-(4-n-Heptylcyclohexen-1-yl)-benzol,
1,4-Bis-(4-n-Octylcyclohexen-1-yl)-benzol.

Bei Verwendung äquivalenter Mengen 4,4'-Dibrombiphenyl anstelle von 1,4-Dibrombenzol werden hergestellt:

4,4'-Bis-(4-Methylcyclohexen-1-yl)-biphenyl,
4,4'-Bis-(4-Ethylcyclohexen-1-yl)-biphenyl,
4,4'-Bis-(4-n-Propylcyclohexen-1-yl)-biphenyl,
4,4'-Bis-(4-n-Butylcyclohexen-1-yl)-biphenyl,
4,4'-Bis-(4-n-Pentylcyclohexen-1-yl)-biphenyl,
4,4'-Bis-(4-n-Hexylcyclohexen-1-yl)-biphenyl,
4,4'-Bis-(4-n-Heptylcyclohexen-1-yl)-biphenyl.

## Beispiel 4

Eine Lösung von 31 g 4-(4-n-Pentylcyclohexen-1-yl)-brombenzol in 50 ml Tetrahydrofuran wird langsam unter Rühren zu 24 g Magnesiumspänen in 50 ml Tetrahydrofuran getropft. Anschließend wird das Reaktionsgemisch 2 Stunden zum Sieden erhitzt und nach dem Abkühlen tropfenweise mit einer Lösung von 14 g 4-n-Propylcyclohexanon in 20 ml Tetrahydrofuran versetzt. Es wird noch 2 Stunden bei 60° nachgerührt und dann durch Zutropfen von 500 ml 5%wäßriger Salzsäure hydrolysiert. Das hydrolysierte Reaktionsgemisch wird viermal mit insgesamt 800 ml Diethylether extrahiert, die Extrakte mit Wasser gewaschen und eingedampft, zuletzt unter vermindertem Druck. Der sirupöse Rückstand wird in 90 ml Aceton gelöst und diese Lösung mit 5 g p-Toluolsulfonsäure 4 Stunden unter Rückfluß zum Sieden erhitzt. Danach wird das Reaktionsgemisch auf −5° abgekühlt, das auskristallisierte 1-(4-n-Pentylcyclohexen-1-yl)-4-(4-n-propylcyclohexen-1-yl)-benzol abfiltriert und aus Isopropylalkohol umkristallisiert; Ausbeute 23,6 g farblose Kristalle, F. 35°, K. 141°.

Analog werden hergestellt:

1-(4-Ethylcyclohexen-1-yl)-4-(4-methylcyclohexen-1-yl)-benzol,
1-(4-n-Propylcyclohexen-1-yl)-4-(4-methylcyclohexen-1-yl)-benzol,
1-(4-n-Butylcyclohexen-1-yl)-4-(4-methylcyclohexen-1-yl)-benzol,
1-(4-n-Pentylcyclohexen-1-yl)-4-(4-methylcyclohexen-1-yl)-benzol,

1-(4-n-Hexylcyclohexen-1-yl)-4-(4-methylcyclohexen-1-yl)-benzol,
1-(4-n-Heptylcyclohexen-1-yl)-4-(4-methylcyclohexen-1-yl)-benzol,
1-(4-n-Octylcyclohexen-1-yl)-4-(4-methylcyclohexen-1-yl)-benzol,
1-(4-n-Nonylcyclohexen-1-yl)-4-(4-methylcyclohexen-1-yl)-benzol,
1-(4-n-Decylcyclohexen-1-yl)-4-(4-methylcyclohexen-1-yl)-benzol,
1-[4-(2-Methylbutyl)-cyclohexen-1-yl]-4-(4-methylcyclohexen-1-yl)-benzol.

1-(4-n-Propylcyclohexen-1-yl)-4-(4-ethylcyclohexen-1-yl)-benzol,
1-(4-n-Butylcyclohexen-1-yl)-4-(4-ethylcyclohexen-1-yl)-benzol,
1-(4-n-Pentylcyclohexen-1-yl)-4-(4-ethylcyclohexen-1-yl)-benzol,
1-(4-n-Hexylcyclohexen-1-yl)-4-(4-ethylcyclohexen-1-yl)-benzol,
1-(4-n-Heptylcyclohexen-1-yl)-4-(4-ethylcyclohexen-1-yl)-benzol,
1-(4-n-Octylcyclohexen-1-yl)-4-(4-ethylcyclohexen-1-yl)-benzol,
1-(4-n-Nonylcyclohexen-1-yl)-4-(4-ethylcyclohexen-1-yl)-benzol,
1-(4-n-Decylcyclohexen-1-yl)-4-(4-ethylcyclohexen-1-yl)-benzol,
1-[4-(2-Methylbutyl)-cyclohexen-1-yl]-4-(4-ethylcyclohexen-1-yl)-benzol,

1-(4-n-Butylcyclohexen-1-yl)-4-(4-propylcyclohexen-1-yl)-benzol,
1-(4-n-Hexylcyclohexen-1-yl)-4-(4-propylcyclohexen-1-yl)-benzol,
1-(4-n-Heptylcyclohexen-1-yl)-4-(4-propylcyclohexen-1-yl)-benzol,
1-(4-n-Octylcyclohexen-1-yl)-4-(4-propylcyclohexen-1-yl)-benzol,
1-(4-n-Nonylcyclohexen-1-yl)-4-(4-propylcyclohexen-1-yl)-benzol,
1-(4-n-Decylcyclohexen-1-yl)-4-(4-propylcyclohexen-1-yl)-benzol,
1-[4-(2-Methylbutyl)-cyclohexen-1-yl]-4-(4-propylcyclohexen-1-yl)-benzol.

1-(4-n-Pentylcyclohexen-1-yl)-4-(4-n-butylcyclohexen-1-yl)-benzol,
1-(4-n-Hexylcyclohexen-1-yl)-4-(4-n-butylcyclohexen-1-yl)-benzol,
1-(4-n-Heptylcyclohexen-1-yl)-4-(4-n-butylcyclohexen-1-yl)-benzol,
1-(4-n-Octylcyclohexen-1-yl)-4-(4-n-butylcyclohexen-1-yl)-benzol,
1-(4-n-Nonylcyclohexen-1-yl)-4-(4-n-butylcyclohexen-1-yl)-benzol,
1-(4-n-Decylcyclohexen-1-yl)-4-(4-n-butylcyclohexen-1-yl)-benzol,
1-[4-(2-Methylbutyl)-cyclohexen-1-yl]-4-(4-n-butylcyclohexen-1-yl)-benzol.

1-(4-n-Hexylcyclohexen-1-yl)-4-(4-n-pentylcyclohexen-1-yl)-benzol,
1-(4-n-Heptylcyclohexen-1-yl)-4-(4-n-pentylcyclohexen-1-yl)-benzol,
1-(4-n-Octylcyclohexen-1-yl)-4-(4-n-pentylcyclohexen-1-yl)-benzol,
1-(4-n-Nonylcyclohexen-1-yl)-4-(4-n-pentylcyclohexen-1-yl)-benzol,
1-(4-n-Decylcyclohexen-1-yl)-4-(4-n-pentylcyclohexen-1-yl)-benzol,
1-[4-(2-Methylbutyl)-cyclohexen-1-yl]-4-(4-n-pentylcyclohexen-1-yl)-benzol.

1-(4-n-Heptylcyclohexen-1-yl)-4-(4-n-hexylcyclohexen-1-yl)-benzol,
1-(4-n-Octylcyclohexen-1-yl)-4-(4-n-hexylcyclohexen-1-yl)-benzol,
1-(4-n-Nonylcyclohexen-1-yl)-4-(4-n-hexylcyclohexen-1-yl)-benzol,
1-(4-n-Decylcyclohexen-1-yl)-4-(4-n-hexylcyclohexen-1-yl)-benzol,
1-[4-(2-Methylbutyl)-cyclohexen-1-yl]-4-(4-n-hexylcyclohexen-1-yl)-benzol.

4-(4-Ethylcyclohexen-1-yl)-4′-(4-methylcyclohexen-1-yl)-biphenyl,
4-(4-n-Propylcyclohexen-1-yl)-4′-(4-methylcyclohexen-1-yl)-biphenyl,
4-(4-n-Butylcyclohexen-1-yl)-4′-(4-methylcyclohexen-1-yl)-biphenyl,
4-(4-n-Pentylcyclohexen-1-yl)-4′-(4-methylcyclohexen-1-yl)-biphenyl,
4-(4-n-Hexylcyclohexen-1-yl)-4′-(4-methylcyclohexen-1-yl)-biphenyl,
4-(4-n-Heptylcyclohexen-1-yl)-4′-(4-methylcyclohexen-1-yl)-biphenyl,
4-(4-n-Octylcyclohexen-1-yl)-4′-(4-methylcyclohexen-1-yl)-biphenyl,
4-(4-n-Nonylcyclohexen-1-yl)-4′-(4-methylcyclohexen-1-yl)-biphenyl,
4-(4-n-Decylcyclohexen-1-yl)-4′-(4-methylcyclohexen-1-yl)-biphenyl,
4-[4-(2-Methylbutyl)-cyclohexen-1-yl]-4′-(4-methylcyclohexen-1-yl)-biphenyl.

4-(4-n-Propylcyclohexen-1-yl)-4′-(4-ethylcyclohexen-1-yl)-biphenyl,
4-(4-n-Butylcyclohexen-1-yl)-4′-(4-ethylcyclohexen-1-yl)-biphenyl,
4-(4-n-Pentylcyclohexen-1-yl)-4′-(4-ethylcyclohexen-1-yl)-biphenyl,
4-(4-n-Hexylcyclohexen-1-yl)-4′-(4-ethylcyclohexen-1-yl)-biphenyl,
4-(4-n-Heptylcyclohexen-1-yl)-4′-(4-ethylcyclohexen-1-yl)-biphenyl,
4-(4-n-Octylcyclohexen-1-yl)-4′-(4-ethylcyclohexen-1-yl)-biphenyl,
4-(4-n-Nonylcyclohexen-1-yl)-4′-(4-ethylcyclohexen-1-yl)-biphenyl,
4-(4-n-Decylcyclohexen-1-yl)-4′-(4-ethylcyclohexen-1-yl)-biphenyl,

4-[4-(2-Methylbutyl)-cyclohexen-1-yl]-4'-(4-ethylcyclohexen-1-yl)-biphenyl.

4-(4-n-Butylcyclohexen-1-yl)-4'-(4-n-propylcyclohexen-1-yl)-biphenyl,
4-(4-n-Pentylcyclohexen-1-yl)-4'-(4-n-propylcyclohexen-1-yl)-biphenyl,
4-(4-n-Hexylcyclohexen-1-yl)-4'-(4-n-propylcyclohexen-1-yl)-biphenyl,
4-(4-n-Heptylcyclohexen-1-yl)-4'-(4-n-propylcyclohexen-1-yl)-biphenyl,
4-(4-n-Octylcyclohexen-1-yl)-4'-(4-n-propylcyclohexen-1-yl)-biphenyl,
4-(4-n-Nonylcyclohexen-1-yl)-4'-(4-n-propylcyclohexen-1-yl)-biphenyl,
4-(4-n-Decylcyclohexen-1-yl)-4'-(4-n-propylcyclohexen-1-yl)-biphenyl,
4-[4-(2-Methylbutyl)-cyclohexen-1-yl]-4'-(4-n-propylcyclohexen-1-yl)-biphenyl.

4-(4-n-Pentylcyclohexen-1-yl)-4'-(4-n-butylcyclohexen-1-yl)-biphenyl,
4-(4-n-Hexylcyclohexen-1-yl)-4'-(4-n-butylcyclohexen-1-yl)-biphenyl,
4-(4-n-Heptylcyclohexen-1-yl)-4'-(4-n-butylcyclohexen-1-yl)-biphenyl,
4-(4-n-Octylcyclohexen-1-yl)-4'-(4-n-butylcyclohexen-1-yl)-biphenyl,
4-(4-n-Nonylcyclohexen-1-yl)-4'-(4-n-butylcyclohexen-1-yl)-biphenyl,
4-(4-n-Decylcyclohexen-1-yl)-4'-(4-n-butylcyclohexen-1-yl)-biphenyl,
4-[4-(2-Methylbutyl)-cyclohexen-1-yl]-4'-(4-n-butylcyclohexen-1-yl)-biphenyl.

4-(4-n-Hexylcyclohexen-1-yl)-4'-(4-n-pentylcyclohexen-1-yl)-biphenyl,
4-(4-n-Heptylcyclohexen-1-yl)-4'-(4-n-pentylcyclohexen-1-yl)-biphenyl,
4-(4-n-Octylcyclohexen-1-yl)-4'-(4-n-pentylcyclohexen-1-yl)-biphenyl,
4-(4-n-Nonylcyclohexen-1-yl)-4'-(4-n-pentylcyclohexen-1-yl)-biphenyl,
4-(4-n-Decylcyclohexen-1-yl)-4'-(4-n-pentylcyclohexen-1-yl)-biphenyl,
4-[4-(2-Methylbutyl)-cyclohexen-1-yl]-4'-(4-n-pentylcyclohexen-1-yl)-biphenyl.

4-(4-n-Heptylcyclohexen-1-yl)-4'-(4-n-hexylcyclohexen-1-yl)-biphenyl,
4-(4-n-Octylcyclohexen-1-yl)-4'-(4-n-hexylcyclohexen-1-yl)-biphenyl,
4-(4-n-Nonylcyclohexen-1-yl)-4'-(4-n-hexylcyclohexen-1-yl)-biphenyl,
4-(4-n-Decylcyclohexen-1-yl)-4'-(4-n-hexylcyclohexen-1-yl)-biphenyl,
4-[4-(2-Methylbutyl)-cyclohexen-1-yl]-4'-(4-n-hexylcyclohexen-1-yl)-biphenyl.

## Beispiel 5

Analog Beispiel 4 werden ausgehend von den entsprechenden 4-(trans-4-Alkylcyclohexyl)-brombenzolen bzw. 4-(trans-4-Alkylcyclohexyl)-4'-brombiphenylen die folgenden erfindungsgemäßen Verbindungen hergestellt:

1-(4-Methylcyclohexen-1-yl)-4-(trans-4-methylcyclohexyl)-benzol,
1-(4-Ethylcyclohexen-1-yl)-4-(trans-4-methylcyclohexyl)-benzol,
1-(4-n-Propylcyclohexen-1-yl)-4-(trans-4-methylcyclohexyl)-benzol,
1-(4-n-Butylcyclohexen-1-yl)-4-(trans-4-methylcyclohexyl)-benzol,
1-(4-n-Pentylcyclohexen-1-yl)-4-(trans-4-methylcyclohexyl)-benzol,
1-(4-n-Hexylcyclohexen-1-yl)-4-(trans-4-methylcyclohexyl)-benzol,
1-(4-n-Heptylcyclohexen-1-yl)-4-(trans-4-methylcyclohexyl)-benzol,
1-(4-n-Octylcyclohexen-1-yl)-4-(trans-4-methylcyclohexyl)-benzol,

1-(4-n-Nonylcyclohexen-1-yl)-4-(trans-4-methylcyclohexyl)-benzol,
1-(4-n-Decylcyclohexen-1-yl)-4-(trans-4-methylcyclohexyl)-benzol,
1-[4-(2-Methylbutyl)-cyclohexen-1-yl]-4-(trans-4-methylcyclohexyl)-benzol.

1-(4-Methylcyclohexen-1-yl)-4-(trans-4-ethylcyclohexyl)-benzol,
1-(4-Ethylcyclohexen-1-yl)-4-(trans-4-ethylcyclohexyl)-benzol,
1-(4-n-Propylcyclohexen-1-yl)-4-(trans-4-ethylcyclohexyl)-benzol,
1-(4-n-Butylcyclohexen-1-yl)-4-(trans-4-ethylcyclohexyl)-benzol,
1-(4-n-Pentylcyclohexen-1-yl)-4-(trans-4-ethylcyclohexyl)-benzol,
1-(4-n-Hexylcyclohexen-1-yl)-4-(trans-4-ethylcyclohexyl)-benzol,
1-(4-n-Heptylcyclohexen-1-yl)-4-(trans-4-ethylcyclohexyl)-benzol,
1-(4-n-Octylcyclohexen-1-yl)-4-(trans-4-ethylcyclohexyl)-benzol,
1-(4-n-Nonylcyclohexen-1-yl)-4-(trans-4-ethylcyclohexyl)-benzol,
1-(4-n-Decylcyclohexen-1-yl)-4-(trans-4-ethylcyclohexyl)-benzol,
1-[4-(2-Methylbutyl)-cyclohexen-1-yl]-4-(trans-4-ethylcyclohexyl)-benzol.

1-(4-Methylcyclohexen-1-yl)-4-(trans-4-n-propylcyclohexyl)-benzol,

1-(4-Ethylcyclohexen-1-yl)-4-(trans-4-n-propylcyclohexyl)-benzol,
1-(4-n-Propylcyclohexen-1-yl)-4-(trans-4-n-propylcyclohexyl)-benzol,
1-(4-n-Butylcyclohexen-1-yl)-4-(trans-4-n-propylcyclohexyl)-benzol,
1-(4-n-Pentylcyclohexen-1-yl)-4-(trans-4-n-propylcyclohexyl)-benzol,
1-(4-n-Hexylcyclohexen-1-yl)-4-(trans-4-n-propylcyclohexyl)-benzol,
1-(4-n-Heptylcyclohexen-1-yl)-4-(trans-4-n-propylcyclohexyl)-benzol,
1-(4-n-Octylcyclohexen-1-yl)-4-(trans-4-n-propylcyclohexyl)-benzol,
1-(4-n-Nonylcyclohexen-1-yl)-4-(trans-4-n-propylcyclohexyl)-benzol,
1-(4-n-Decylcyclohexen-1-yl)-4-(trans-4-n-propylcyclohexyl)-benzol,
1-[4-(2-Methylbutyl)-cyclohexen-1-yl]-4-(trans-4-n-propylcyclohexyl)-benzol.

1-(4-Methylcyclohexen-1-yl)-4-(trans-4-n-butylcyclohexyl)-benzol,
1-(4-Ethylcyclohexen-1-yl)-4-(trans-4-n-butylcyclohexyl)-benzol,
1-(4-n-Propylcyclohexen-1-yl)-4-(trans-4-n-butylcyclohexyl)-benzol,
1-(4-n-Butylcyclohexen-1-yl)-4-(trans-4-n-butylcyclohexyl)-benzol,
1-(4-n-Pentylcyclohexen-1-yl)-4-(trans-4-n-butylcyclohexyl)-benzol,
1-(4-n-Hexylcyclohexen-1-yl)-4-(trans-4-n-butylcyclohexyl)-benzol,
1-(4-n-Heptylcyclohexen-1-yl)-4-(trans-4-n-butylcyclohexyl)-benzol,
1-(4-n-Octylcyclohexen-1-yl)-4-(trans-4-n-butylcyclohexyl)-benzol,
1-(4-n-Nonylcyclohexen-1-yl)-4-(trans-4-n-butylcyclohexyl)-benzol,
1-(4-n-Decylcyclohexen-1-yl)-4-(trans-4-n-butylcyclohexyl)-benzol,
1-[4-(2-Methylbutyl)-cyclohexen-1-yl]-4-(trans-4-n-butylcyclohexyl)-benzol.

1-(4-Methylcyclohexen-1-yl)-4-(trans-4-n-pentylcyclohexyl)-benzol,
1-(4-Ethylcyclohexen-1-yl)-4-(trans-4-n-pentylcyclohexyl)-benzol,
1-(4-n-Propylcyclohexen-1-yl)-4-(trans-4-n-pentylcyclohexyl)-benzol,
1-(4-n-Butylcyclohexen-1-yl)-4-(trans-4-n-pentylcyclohexyl)-benzol,
1-(4-n-Pentylcyclohexen-1-yl)-4-(trans-4-n-pentylcyclohexyl)-benzol,
1-(4-n-Hexylcyclohexen-1-yl)-4-(trans-4-n-pentylcyclohexyl)-benzol,
1-(4-n-Heptylcyclohexen-1-yl)-4-(trans-4-n-pentylcyclohexyl)-benzol,
1-(4-n-Octylcyclohexen-1-yl)-4-(trans-4-n-pentylcyclohexyl)-benzol,
1-(4-n-Nonylcyclohexen-1-yl)-4-(trans-4-n-pentylcyclohexyl)-benzol,
1-(4-n-Decylcyclohexen-1-yl)-4-(trans-4-n-pentylcyclohexyl)-benzol,
1-[4-(2-Methylbutyl)-cyclohexen-1-yl]-4-(trans-4-n-pentylcyclohexyl)-benzol.

1-(4-Methylcyclohexen-1-yl)-4-(trans-4-n-hexylcyclohexyl)-benzol,
1-(4-Ethylcyclohexen-1-yl)-4-(trans-4-n-hexylcyclohexyl)-benzol,
1-(4-n-Propylcyclohexen-1-yl)-4-(trans-4-n-hexylcyclohexyl)-benzol,
1-(4-n-Butylcyclohexen-1-yl)-4-(trans-4-n-hexylcyclohexyl)-benzol,
1-(4-n-Pentylcyclohexen-1-yl)-4-(trans-4-n-hexylcyclohexyl)-benzol,
1-(4-n-Hexylcyclohexen-1-yl)-4-(trans-4-n-hexylcyclohexyl)-benzol,
1-(4-n-Heptylcyclohexen-1-yl)-4-(trans-4-n-hexylcyclohexyl)-benzol,
1-(4-n-Octylcyclohexen-1-yl)-4-(trans-4-n-hexylcyclohexyl)-benzol,
1-(4-n-Nonylcyclohexen-1-yl)-4-(trans-4-n-hexylcyclohexyl)-benzol,
1-(4-n-Decylcyclohexen-1-yl)-4-(trans-4-n-hexylcyclohexyl)-benzol,
1-[4-(2-Methylbutyl)-cyclohexen-1-yl]-4-(trans-4-n-hexylcyclohexyl)-benzol.

1-(4-Methylcyclohexen-1-yl)-4-(trans-4-n-heptylcyclohexyl)-benzol,
1-(4-Ethylcyclohexen-1-yl)-4-(trans-4-n-heptylcyclohexyl)-benzol,
1-(4-n-Propylcyclohexen-1-yl)-4-(trans-4-n-heptylcyclohexyl)-benzol,
1-(4-n-Butylcyclohexen-1-yl)-4-(trans-4-n-heptylcyclohexyl)-benzol,
1-(4-n-Pentylcyclohexen-1-yl)-4-(trans-4-n-heptylcyclohexyl)-benzol,
1-(4-n-Hexylcyclohexen-1-yl)-4-(trans-4-n-heptylcyclohexyl)-benzol,
1-(4-n-Heptylcyclohexen-1-yl)-4-(trans-4-n-heptylcyclohexyl)-benzol,
1-(4-n-Octylcyclohexen-1-yl)-4-(trans-4-n-heptylcyclohexyl)-benzol,
1-[4-(2-Methylbutyl)-cyclohexen-1-yl]-4-(trans-4-n-heptylcyclohexyl)-benzol.

1-(4-Methylcyclohexen-1-yl)-4-(trans-4-n-octylcyclohexyl)-benzol,
1-(4-Ethylcyclohexen-1-yl)-4-(trans-4-n-octylcyclohexyl)-benzol,
1-(4-n-Propylcyclohexen-1-yl)-4-(trans-4-n-octylcyclohexyl)-benzol,
1-(4-n-Butylcyclohexen-1-yl)-4-(trans-4-n-octylcyclohexyl)-benzol,
1-(4-n-Pentylcyclohexen-1-yl)-4-(trans-4-n-octylcyclohexyl)-benzol,
1-(4-n-Hexylcyclohexen-1-yl)-4-(trans-4-n-octylcyclohexyl)-benzol,
1-(4-n-Heptylcyclohexen-1-yl)-4-(trans-4-n-octylcyclohexyl)-benzol,
1-(4-n-Octylcyclohexen-1-yl)-4-(trans-4-n-octylcyclohexyl)-benzol,

1-[4-(2-Methylbutyl)-cyclohexen-1-yl]-4-(trans-4-n-octylcyclohexyl)-benzol.

1-(4-Methylcyclohexen-1-yl)-4-(trans-4-n-nonylcyclohexyl)-benzol,
1-(4-Ethylcyclohexen-1-yl)-4-(trans-4-n-nonylcyclohexyl)-benzol,
1-(4-n-Propylcyclohexen-1-yl)-4-(trans-4-n-nonylcyclohexyl)-benzol,
1-(4-n-Butylcyclohexen-1-yl)-4-(trans-4-n-nonylcyclohexyl)-benzol,
1-(4-n-Pentylcyclohexen-1-yl)-4-(trans-4-n-nonylcyclohexyl)-benzol,
1-(4-n-Hexylcyclohexen-1-yl)-4-(trans-4-n-nonylcyclohexyl)-benzol.

1-(4-Methylcyclohexen-1-yl)-4-(trans-4-n-decylcyclohexyl)-benzol,
1-(4-Ethylcyclohexen-1-yl)-4-(trans-4-n-decylcyclohexyl)-benzol,
1-(4-n-Propylcyclohexen-1-yl)-4-(trans-4-n-decylcyclohexyl)-benzol,
1-(4-n-Butylcyclohexen-1-yl)-4-(trans-4-n-decylcyclohexyl)-benzol,
1-(4-n-Pentylcyclohexen-1-yl)-4-(trans-4-n-decylcyclohexyl)-benzol,
1-(4-n-Hexylcyclohexen-1-yl)-4-(trans-4-n-decylcyclohexyl)-benzol.

4-(4-Methylcyclohexen-1-yl)-4'-(trans-4-methylcyclohexyl)-biphenyl,
4-(4-Ethylcyclohexen-1-yl)-4'-(trans-4-methylcyclohexyl)-biphenyl,
4-(4-n-Propylcyclohexen-1-yl)-4'-(trans-4-methylcyclohexyl)-biphenyl,
4-(4-n-Butylcyclohexen-1-yl)-4'-(trans-4-methylcyclohexyl)-biphenyl,
4-(4-n-Pentylcyclohexen-1-yl)-4'-(trans-4-methylcyclohexyl)-biphenyl,
4-(4-n-Hexylcyclohexen-1-yl)-4'-(trans-4-methylcyclohexyl)-biphenyl,
4-(4-n-Heptylcyclohexen-1-yl)-4'-(trans-4-methylcyclohexyl)-biphenyl,
4-(4-n-Octylcyclohexen-1-yl)-4'-(trans-4-methylcyclohexyl)-biphenyl,
4-(4-n-Nonylcyclohexen-1-yl)-4'-(trans-4-methylcyclohexyl)-biphenyl,
4-(4-n-Decylcyclohexen-1-yl)-4'-(trans-4-methylcyclohexyl)-biphenyl,
4-[4-(2-Methylbutyl)-cyclohexen-1-yl]-4'-(trans-4-methylcyclohexyl)-biphenyl.

4-(4-Methylcyclohexen-1-yl)-4'-(trans-4-ethylcyclohexyl)-biphenyl,
4-(4-Ethylcyclohexen-1-yl)-4'-(trans-4-ethylcyclohexyl)-biphenyl,
4-(4-n-Propylcyclohexen-1-yl)-4'-(trans-4-ethylcyclohexyl)-biphenyl,
4-(4-n-Butylcyclohexen-1-yl)-4'-(trans-4-ethylcyclohexyl)-biphenyl,
4-(4-n-Pentylcyclohexen-1-yl)-4'-(trans-4-ethylcyclohexyl)-biphenyl,
4-(4-n-Hexylcyclohexen-1-yl)-4'-(trans-4-ethylcyclohexyl)-biphenyl,
4-(4-n-Heptylcyclohexen-1-yl)-4'-(trans-4-ethylcyclohexyl)-biphenyl,
4-(4-n-Octylcyclohexen-1-yl)-4'-(trans-4-ethylcyclohexyl)-biphenyl,
4-(4-n-Nonylcyclohexen-1-yl)-4'-(trans-4-ethylcyclohexyl)-biphenyl,
4-(4-n-Decylcyclohexen-1-yl)-4'-(trans-4-ethylcyclohexyl)-biphenyl,
4-[4-(2-Methylbutyl)-cyclohexen-1-yl]-4'-(trans-4-ethylcyclohexyl)-biphenyl.

4-(4-Methylcyclohexen-1-yl)-4'-(trans-4-n-propylcyclohexyl)-biphenyl,
4-(4-Ethylcyclohexen-1-yl)-4'-(trans-4-n-propylcyclohexyl)-biphenyl,
4-(4-n-Propylcyclohexen-1-yl)-4'-(trans-4-n-propylcyclohexyl)-biphenyl,
4-(4-n-Butylcyclohexen-1-yl)-4'-(trans-4-n-propylcyclohexyl)-biphenyl,
4-(4-n-Pentylcyclohexen-1-yl)-4'-(trans-4-n-propylcyclohexyl)-biphenyl,
4-(4-n-Hexylcyclohexen-1-yl)-4'-(trans-4-n-propylcyclohexyl)-biphenyl,
4-(4-n-Heptylcyclohexen-1-yl)-4'-(trans-4-n-propylcyclohexyl)-biphenyl,
4-(4-n-Octylcyclohexen-1-yl)-4'-(trans-4-n-propylcyclohexyl)-biphenyl,
4-(4-n-Nonylcyclohexen-1-yl)-4'-(trans-4-n-propylcyclohexyl)-biphenyl,
4-(4-n-Decylcyclohexen-1-yl)-4'-(trans-4-n-propylcyclohexyl)-biphenyl,
4-[4-(2-Methylbutyl)-cyclohexen-1-yl]-4'-(trans-4-n-propylcyclohexyl)-biphenyl.

4-(4-Methylcyclohexen-1-yl)-4'-(trans-4-n-butylcyclohexyl)-biphenyl,
4-(4-Ethylcyclohexen-1-yl)-4'-(trans-4-n-butylcyclohexyl)-biphenyl,
4-(4-n-Propylcyclohexen-1-yl)-4'-(trans-4-n-butylcyclohexyl)-biphenyl,
4-(4-n-Butylcyclohexen-1-yl)-4'-(trans-4-n-butylcyclohexyl)-biphenyl,
4-(4-n-Pentylcyclohexen-1-yl)-4'-(trans-4-n-butylcyclohexyl)-biphenyl,
4-(4-n-Hexylcyclohexen-1-yl)-4'-(trans-4-n-butylcyclohexyl)-biphenyl,
4-(4-n-Heptylcyclohexen-1-yl)-4'-(trans-4-n-butylcyclohexyl)-biphenyl,
4-(4-n-Octylcyclohexen-1-yl)-4'-(trans-4-n-butylcyclohexyl)-biphenyl,
4-(4-n-Nonylcyclohexen-1-yl)-4'-(trans-4-n-butylcyclohexyl)-biphenyl,
4-(4-n-Decylcyclohexen-1-yl)-4'-(trans-4-n-butylcyclohexyl)-biphenyl,
4-[4-(3-Methylbutyl)-cyclohexen-1-yl]-4'-(trans-4-n-butylcyclohexyl)-biphenyl.

4-(4-Methylcyclohexen-1-yl)-4'-(trans-4-n-pentylcyclohexyl)-biphenyl,

4-(4-Ethylcyclohexen-1-yl)-4'-(trans-4-n-pentylcyclohexyl)-biphenyl,
4-(4-n-Propylcyclohexen-1-yl)-4'-(trans-4-n-pentylcyclohexyl)-biphenyl,

4-(4-n-Butylcyclohexen-1-yl)-4'-(trans-4-n-pentylcyclohexyl)-biphenyl,
4-(4-n-Pentylcyclohexen-1-yl)-4'-(trans-4-n-pentylcyclohexyl)-biphenyl,
4-(4-n-Hexylcyclohexen-1-yl)-4'-(trans-4-n-pentylcyclohexyl)-biphenyl,
4-(4-n-Heptylcyclohexen-1-yl)-4'-(trans-4-n-pentylcyclohexyl)-biphenyl,
4-(4-n-Octylcyclohexen-1-yl)-4'-(trans-4-n-pentylcyclohexyl)-biphenyl,
4-(4-n-Nonylcyclohexen-1-yl)-4'-(trans-4-n-pentylcyclohexyl)-biphenyl,
4-[4-(2-Methylbutyl)-cyclohexen-1-yl]-4'-(trans-4-n-pentylcyclohexyl)-biphenyl.

4-(4-Methylcyclohexen-1-yl)-4'-(trans-4-n-hexylcyclohexyl)-biphenyl,
4-(4-Ethylcyclohexen-1-yl)-4'-(trans-4-n-hexylcyclohexyl)-biphenyl,
4-(4-n-Propylcyclohexen-1-yl)-4'-(trans-4-n-hexylcyclohexyl)-biphenyl,
4-(4-n-Butylcyclohexen-1-yl)-4'-(trans-4-n-hexylcyclohexyl)-biphenyl,
4-(4-n-Pentylcyclohexen-1-yl)-4'-(trans-4-n-hexylcyclohexyl)-biphenyl,
4-(4-n-Hexylcyclohexen-1-yl)-4'-(trans-4-n-hexylcyclohexyl)-biphenyl,
4-(4-n-Heptylcyclohexen-1-yl)-4'-(trans-4-n-hexylcyclohexyl)-biphenyl,
4-(4-n-Octylcyclohexen-1-yl)-4'-(trans-4-n-hexylcyclohexyl)-biphenyl,
4-(4-n-Nonylcyclohexen-1-yl)-4'-(trans-4-n-hexylcyclohexyl)-biphenyl,
4-(4-n-Decylcyclohexen-1-yl)-4'-(trans-4-n-hexylcyclohexyl)-biphenyl,
4-[4-(2-Methylbutyl)-cyclohexen-1-yl]-4'-(trans-4-n-hexylcyclohexyl)-biphenyl.

4-(4-Methylcyclohexen-1-yl)-4'-(trans-4-n-heptylcyclohexyl)-biphenyl,
4-(4-Ethylcyclohexen-1-yl)-4'-(trans-4-n-heptylcyclohexyl)-biphenyl,
4-(4-n-Propylcyclohexen-1-yl)-4'-(trans-4-n-heptylcyclohexyl)-biphenyl,
4-(4-n-Butylcyclohexen-1-yl)-4'-(trans-4-n-heptylcyclohexyl)-biphenyl,
4-(4-n-Pentylcyclohexen-1-yl)-4'-(trans-4-n-heptylcyclohexyl)-biphenyl,
4-(4-n-Hexylcyclohexen-1-yl)-4'-(trans-4-n-heptylcyclohexyl)-biphenyl,
4-(4-n-Heptylcyclohexen-1-yl)-4'-(trans-4-n-heptylcyclohexyl)-biphenyl,
4-[4-(2-Methylbutyl)-cyclohexen-1-yl]-4'-(trans-4-n-heptylcyclohexyl)-biphenyl.

4-(4-Methylcyclohexen-1-yl)-4'-(trans-4-n-octylcyclohexyl)-biphenyl,
4-(4-Ethylcyclohexen-1-yl)-4'-(trans-4-n-octylcyclohexyl)-biphenyl,
4-(4-n-Propylcyclohexen-1-yl)-4'-(trans-4-n-octylcyclohexyl)-biphenyl,
4-(4-n-Butylcyclohexen-1-yl)-4'-(trans-4-n-octylcyclohexyl)-biphenyl,
4-(4-n-Pentylcyclohexen-1-yl)-4'-(trans-4-n-octylcyclohexyl)-biphenyl,
4-(4-n-Hexylcyclohexen-1-yl)-4'-(trans-4-n-octylcyclohexyl)-biphenyl,
4-[4-(2-Methylbutyl)-cyclohexen-1-yl]-4'-(trans-4-n-octylcyclohexyl)-biphenyl.

4-(4-Methylcyclohexen-1-yl)-4'-(trans-4-n-nonylcyclohexyl)-biphenyl,
4-(4-Ethylcyclohexen-1-yl)-4'-(trans-4-n-nonylcyclohexyl)-biphenyl,
4-(4-n-Propylcyclohexen-1-yl)-4'-(trans-4-n-nonylcyclohexyl)-biphenyl,
4-(4-n-Butylcyclohexen-1-yl)-4'-(trans-4-n-nonylcyclohexyl)-biphenyl,
4-(4-n-Pentylcyclohexen-1-yl)-4'-(trans-4-n-nonylcyclohexyl)-biphenyl,
4-(4-n-Hexylcyclohexen-1-yl)-4'-(trans-4-n-nonylcyclohexyl)-biphenyl.

4-(4-Methylcyclohexen-1-yl)-4'-(trans-4-n-decylcyclohexyl)-biphenyl,
4-(4-Ethylcyclohexen-1-yl)-4'-(trans-4-n-decylcyclohexyl)-biphenyl,
4-(4-n-Propylcyclohexen-1-yl)-4'-(trans-4-n-decylcyclohexyl)-biphenyl,
4-(4-n-Butylcyclohexen-1-yl)-4'-(trans-4-n-decylcyclohexyl)-biphenyl,
4-(4-n-Pentylcyclohexen-1-yl)-4'-(trans-4-n-decylcyclohexyl)-biphenyl,
4-(4-n-Hexylcyclohexen-1-yl)-4'-(trans-4-n-decylcyclohexyl)-biphenyl.

Die folgenden Beispiele betreffen die Verwendung der erfindungsgemäßen partiell hydrierten Oligo-1,4-phenyle als Komponenten flüssigkristalliner Dielektrika.

## Beispiel 6

a) Ein Dielektrikum

33,3% 4-n-Butyl-4'-methoxy-azoxybenzol,
16,7% 4-Ethyl-4'-methoxy-azoxybenzol und
50,0% 4-(trans-4-n-Pentylcyclohexyl)-benzonitril

hat einen Klärpunkt von 64°, eine Schwellenspannung in der verdrillten nematischen Zelle von 1,7 V bei 40° und eine Temperaturabhängigkeit der Schwellenspannung von 10,5 mV pro Grad Celsius.

b) Ein Dielektrikum aus 86% der vorstehend beschriebenen Mischung und 14% der erfindungsgemäßen Verbindung 4-(trans-4-n-Pentylcyclohexyl)-4'-(trans-4-n-hexylcyclohexyl)-biphenyl hat demgegenüber einen Klärpunkt von 80°, eine Schwellenspannung in der verdrillten nematischen Zelle von 1,9 V bei 40° und eine Temperaturabhängigkeit der Schwellenspannung von nur 4,7 mV pro Grad Celcius.

Beispiel 7

a) Ein Dielektrikum aus

12% 4-(trans-4-Ethylcyclohexyl)-benzonitril,
23% 4-(trans-4-n-Butylcyclohexyl)-benzonitril,
14% 4-Ethyl-4'-cyanobiphenyl,
10% 4-n-Propyloxy-4'-cyanobiphenyl,
20% 4-(trans-4-Ethylcyclohexyl)-benzoesäure-(trans-4-n-propylcyclohexyl)-ester und
21% 4-(trans-4-n-Butylcyclohexyl)-benzoesäure-(trans-4-n-propylcyclohexyl)-ester

hat einen F. von −5°, einen K. von 68°, eine Schwellenspannung in der verdrillten nematischen Zelle von 1,52 V bei 40° und eine Temperaturabhängigkeit der Schwellenspannung von 6,0 mV pro Grad Celsius.

b) Ein Dielektrikum aus 90% der vorstehend beschriebenen Mischung und 10% der erfindungsgemäßen Verbindung 4-(trans-4-n-Pentylcyclohexyl)-4'-(trans-4-n-hexylcyclohexyl)-biphenyl hat demgegenüber einen F. von −6°, einen K. von 81°, eine Schwellenspannung in der verdrillten nematischen Zelle von 1,74 V bei 40° und eine Temperaturabhängigkeit der Schwellenspannung von nur 3,0 mV pro Grad Celsius.

Beispiel 8

a) Ein Dielektrikum aus

24% 4-(trans-4-n-Propylcyclohexyl)-benzonitril,
36% 4-(trans-4-n-Pentylcyclohexyl)-benzonitril,
25% 4-(trans-4-n-Heptylcyclohexyl)-benzonitril und
15% 4-(trans-4-n-Pentylcyclohexyl)-4'-cyanobiphenyl

hat einen F. von −6°, einen K. von 72°, eine Schwellenspannung in der verdrillten nematischen Zelle von 1,7 V bei 40° und eine Temperaturabhängigkeit der Schwellenspannung von 10,0 mV pro Grad Celsius.

b) Ein Dielektrikum aus 90% der vorstehend beschriebenen Mischung und 10% der erfindungsgemäßen Verbindung 4,4-Bis-(trans-4-n-Pentylcyclohexyl)-biphenyl hat demgegenüber einen F. von −7°, einen K. von 88°, eine Schwellenspannung in der verdrillten nematischen Zelle von 2,0 V bei 40° und eine Temperaturabhängigkeit der Schwellenspannung von nur 7,5 mV pro Grad Celsius.

Beispiel 9

Ein Dielektrikum aus

28% Anissäure-4-n-pentylphenylester,
22% 4-n-Hexanoyloxybenzoesäure-4-n-propylphenylester,
20% 4-(trans-4-n-Butylcyclohexyl)-benzoesäure-4-n-propylcyclohexyl-ester,
19% 4-(trans-4-n-Propylcyclohexyl)-ethylbenzol und
11% 4-(trans-4-n-Pentylcyclohexyl)-4'-(trans-4-n-propylcyclohexyl)-biphenyl

hat einen F. von −7°, einen K. von +70°, eine Viskosität von $35 \cdot 10^{-3}$ Pa · s bei 20° und eine dielektrische Anisotropie von −0,2. Es ist damit hervorragend für Anzeigeelemente geeignet, die nach dem Prinzip der dynamische Streuung arbeiten.

Beispiel 10

Ein Dielektrikum aus

23% 4-(trans-4-n-Pentylcyclohexyl)-benzonitril,
17% 4-(trans-4-n-Propylcyclohexyl)-benzonitril,
16% 4-(trans-4-n-Propylcyclohexyl)-phenetol,
12% 4-(trans-4-n-Propylcyclohexyl)-1-n-butyloxybenzol,
22% 4-(trans-4-n-Pentylcyclohexyl)-4'-ethylbiphenyl und
10% 4-(trans-4-n-Propylcyclohexyl)-4'-(trans-4-n-pentylcyclohexyl)-biphenyl

hat einen F. von −20°, einen K. von +85°, eine Viskosität von 19 · $10^{-3}$ Pa · s bei 20°, eine dielektrische Anisotropie von +6,0, eine Schwellenspannung in der verdrillten nematischen Zelle von 2,3 V bei 20° und eine Temperaturabhängigkeit der Schwellenspannung von nur 8 mV pro Grad Celsius. Aufgrund seiner niedrigen Viskosität und des breiten Temperaturbereichs der nematischen Phase ist es für Kraftfahrzeug-Anzeigeelemente besonders gut geeignet.

Beispiel 11

Ein Dielektrikum aus

49% 4-(trans-4-n-Propylcyclohexyl)-1-n-butyryloxybenzol,
21% 4-(trans-4-n-Propylcyclohexyl)-phenetol,
18% 4-(trans-4-n-Propylcyclohexyl)-1-n-butyloxybenzol und
12% 4-(trans-4-n-Propylcyclohexyl)-4'-(trans-4-n-pentylcyclohexyl)-biphenyl

hat eine nematische Phase im Temperaturbereich von −10° bis +59°, eine Viskosität von nur 13 · $10^{-3}$ Pa · s bei 20° und eine dielektrische Anisotropie von −0,59. Es ist damit besonders gut für mit relativ niedrigen Steuerspannungen betriebene, schnell schaltende Anzeigeelemente geeignet, die nach dem Prinzip der dynamischen Streuung arbeiten.

Beispiel 12

Ein Dielektrikum aus

21% 4-(trans-4-n-Pentylcyclohexyl)-benzonitril,
14% 4-(trans-4-n-Propylcyclohexyl)-benzonitril,
16% 4-(trans-4-n-Propylcyclohexyl)-phenetol,
12% 4-(trans-4-n-Propylcyclohexyl)-1-n-butyloxybenzol,
22% 4-(trans-4-n-Pentylcyclohexyl)-4'-ethylbiphenyl,
5% 4-(trans-4-n-Pentylcyclohexyl)-4'-cyanobiphenyl und
10% 4-(trans-4-n-Pentylcyclohexyl)-4'-(trans-4-n-propylcyclohexyl)-biphenyl

hat eine nematische Phase im Temperaturbereich von −20° bis +93°, eine Viskosität von 22 · $10^{-3}$ Pa · s bei 20°, eine dielektrische Anisotropie von +5,7 und eine Schwellenspannung von 2,3 V. Es ist besonders gut zur Verwendung in Kraftfahrzeug-Anzeigeelementen geeignet.

Beispiel 13

Ein Dielektrikum aus

20% 4-(trans-4-n-Butylcyclohexyl)-benzonitril,
18% 4-(trans-4-n-Pentylcyclohexyl)-benzonitril,
17% 4-n-Butyl-4'-cyanobiphenyl,
13% 4-Ethyl-4'-cyanobiphenyl,
12% 4-(trans-4-n-Propylcyclohexyl)-benzoesäure-4-n-propylphenylester,
10% 4-(trans-4-n-Pentylcyclohexyl)-4'-ethylbiphenyl und
10% 4-(trans-4-n-Pentylcyclohexyl)-4'-(trans-4-n-propylcyclohexyl)-biphenyl

hat eine nematische Phase im Temperaturbereich von −4° bis +84°, eine Viskosität von 30 · $10^{-3}$ Pa · s bei 20°, eine dielektrische Anisotropie von +10, eine Schwellenspannung von 1,5 V bei 20° und eine Temperaturabhängigkeit der Schwellenspannung von nur 4 mV pro Grad Celsius. Es ist gut geeignet für

Anzeigeelemente mit hoher Informationsdichte auf der Basis der verdrillten nematischen Zelle, die als Matrixanzeigeelemente für Multiplexbetrieb gebaut sind.

Beispiel 14

Ein Dielektrikum aus

20% 4-(trans-4-n-Propylcyclohexyl)-benzonitril,
16% 4-(trans-4-n-Butylcyclohexyl)-benzonitril,
22% 4-(trans-4-n-Pentylcyclohexyl)-benzonitril,
25% 4-(trans-4-n-Pentylcyclohexyl)-4'-ethylbiphenyl,
7% 4-(trans-4-n-Propylcyclohexyl)-ethylbenzol und
10% 4-(trans-4-n-Pentylcyclohexyl)-4'-(trans-4-n-propylcyclohexyl)-biphenyl

hat eine nematische Phase im Temperaturbereich von −15° bis +85°, eine Viskosität von $21 \cdot 10^{-3}$ Pa · s bei 20°, eine dielektrische Anisotropie von +7,5 und eine Schwellenspannung von 1,8 V bei 20°, die sich um 7,0 mV pro Grad Celsius ändert.

Beispiel 15

Ein Dielektrikum aus

16% 4-(trans-4-Ethylcyclohexyl)-benzonitril,
22% 4-(trans-4-n-Propylcyclohexyl)-benzonitril,
17% 4-(trans-4-n-Butylcyclohexyl)-benzonitril,
27% 4-(trans-4-n-Pentylcyclohexyl)-benzonitril,
8% 4-(trans-4-n-Pentylcyclohexyl)-4'-ethylbiphenyl und
10% 4-(trans-4-n-Pentylcyclohexyl)-4'-(trans-4-n-propylcyclohexyl)-biphenyl

hat eine nematische Phase im Temperaturbereich von −10° bis +66°, eine Viskosität von $23 \cdot 10^{-3}$ Pa · s bei 20° und eine dielektrische Anisotropie von +9,7. Die Schwellenspannung in der verdrillten nematischen Zelle ist 1,6 V. Anzeigeelemente mit diesem Dielektrikum sind beispielsweise für tragbare Rechengeräte gut geeignet.

**Patentansprüche für die Vertragsstaaten: CH, DE, FR, GB, IT, LI, NL**

1. Partiell hydrierte Oligo-1,4-phenylene der Formel I

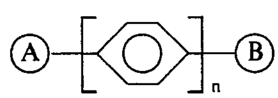

(I)

worin n 1 oder 2 ist und die Ringe A und B, die gleich oder verschieden sind, trans-4-Alkylcyclohexyl oder 4-Alkylcyclohexen-1-yl bedeuten, wobei die Alkylgruppen jeweils bis zu 10 Kohlenstoffatome enthalten.

2. Partiell hydrierte Oligo-1,4-phenylene nach Anspruch 1 der Formel (Ia)

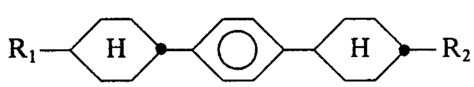

(Ia)

worin $R_1$ und $R_2$ gleich oder verschieden sind und Alkylgruppen mit bis zu 10 Kohlenstoffatomen bedeuten.

3. Partiell hydrierte Oligo-1,4-phenylene nach Anspruch 1 der Formel (Ib)

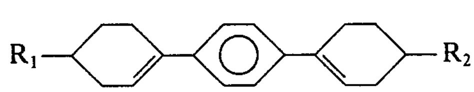

(Ib)

worin $R_1$ und $R_2$ gleich oder verschieden sind und Alkylgruppen mit bis zu 10 Kohlenstoffatomen bedeuten.

4. Partiell hydrierte Oligo-1,4-phenylene nach Anspruch 1 der Formel (Ic)

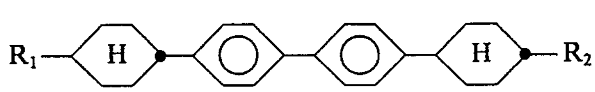

(Ic)

worin $R_1$ und $R_2$ gleich oder verschieden sind und Alkylgruppen mit bis zu 10 Kohlenstoffatomen bedeuten.

5. Partiell hydrierte Oligo-1,4-phenylene nach Anspruch 1 der Formel (Id)

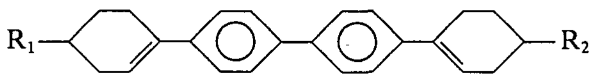 (Id)

worin $R_1$ und $R_2$ gleich oder verschieden sind und Alkylgruppen mit bis zu 10 Kohlenstoffatomen bedeuten.

6. Verwendung der partiell hydrierten Oligo-1,4-phenylene der Formel (I)

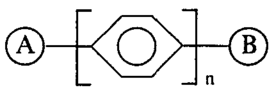 (I)

worin n 1 oder 2 ist und die Ringe A und B, die gleich oder verschieden sind, trans-4-Alkylcyclohexyl oder 4-Alkylcyclohexen-1-yl bedeuten, wobei die Alkylgruppen jeweils bis zu 10 Kohlenstoffatome enthalten, als Komponenten flüssigkristalliner Dielektrika.

7. Flüssigkristallines Dielektrikum für elektrooptische Anzeigeelemente, dadurch gekennzeichnet, daß es mindestens ein partiell hydriertes Oligo-1,4-phenylen der Formel (I) enthält,

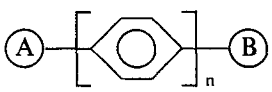 (I)

worin n 1 oder 2 ist und die Ringe A und B, die gleich oder verschieden sind, trans-4-Alkylcyclohexyl oder 4-Alkylcyclohexen-1-yl bedeuten, wobei die Alkylgruppen jeweils bis zu 10 Kohlenstoffatomen enthalten.

8. Elektrooptisches Anzeigeelement auf der Basis einer Flüssigkristallzelle, dadurch gekennzeichnet, daß es ein flüssigkristallines Dielektrikum nach Anspruch 7 enthält.

**Patentansprüche für den Vertragsstaat: AT**

1. Verwendung partiell hydrierter Oligo-1,4-phenylene der Formel (I)

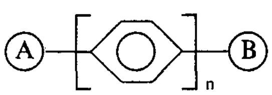 (I)

worin n 1 oder 2 ist und die Ringe A und B, die gleich oder verschieden sind, trans-4-Alkylcyclohexyl oder 4-Alkylcyclohexen-1-yl bedeuten, wobei die Alkylgruppen jeweils bis zu 10 Kohlenstoffatome enthalten, als Komponenten flüssigkristalliner Dielektrika.

2. Flüssigkristallines Dielektrikum für elektrooptische Anzeigeelemente, dadurch gekennzeichnet, daß es mindestens ein partiell hydriertes Oligo-1,4-phenylen der Formel (I) enthält,

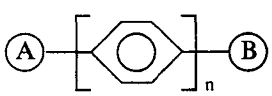 (I)

worin n 1 oder 2 ist und die Ringe A und B, die gleich oder verschieden sind, trans-4-Alkylcyclohexyl oder 4-Alkylcyclohexen-1-yl bedeuten, wobei die Alkylgruppen jeweils bis zu 10 Kohlenstoffatome enthalten.

3. Elektrooptisches Anzeigeelement auf der Basis einer Flüssigkristallzelle, dadurch gekennzeichnet, daß es ein flüssigkristallines Dielektrikum nach Anspruch 7 enthält.

**Claims for the contracting states: CH, DE, FR, GB, IT, LI, NL**

1. Partially hydrogenated oligo-1,4-phenylenes of the formula (I)

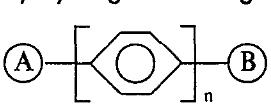 (I)

wherein n is 1 or 2 and the rings A and B, which are identical or different, are trans-4-alkylcyclohexyl or 4-alkylcyclohex-1-enyl, the alkyl groups in each case containing up to 10 carbon atoms.

2. Partially hydrogenated oligo-1,4-phenylenes according to Claim 1 of the formula (Ia)

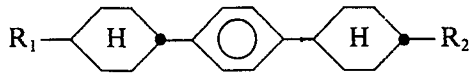
(Ia)

wherein $R_1$ and $R_2$ are identical or different and are alkyl groups with up to 10 carbon atoms.

3. Partiell hydrogenated oligo-1,4-phenylenes according to Claim 1, of the formula (Ib)

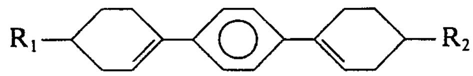
(Ib)

wherein $R_1$ and $R_2$ are identical or different and are alkyl groups with up to 10 carbon atoms.

4. Partially hydrogenated oligo-1,4-phenylenes according to Claim 1, of the formula (Ic)

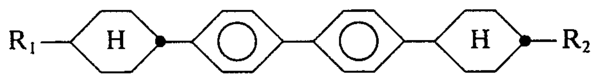
(Ic)

wherein $R_1$ and $R_2$ are identical of different and are alkyl groups with up to 10 carbon atoms.

5. Partially hydrogenated oligo-1,4-phenylenes according to Claim 1, of the formula (Id)

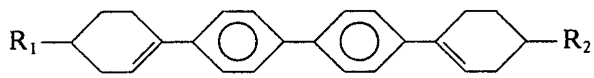
(Id)

wherein $R_1$ and $R_2$ are identical or different and are alkyl groups with up to 10 carbon atoms.

6. Use of the partially hydrogenated oligo-1,4-phenylenes of the formula (I)

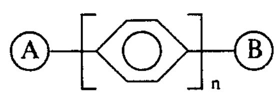
(I)

wherein n ist 1 or 2 and the rings A and B, which are identical or different, are trans-4-alkylcyclohexyl or 4-alkylcyclohex-1-enyl, the alkyl groups in each case containing up to 10 carbon atoms, as components of liquid crystal dielectrics.

7. Liquid crystal dielectric for electro-optical display elements, characterised in that it contains at least one partially hydrogenated oligo-1,4-phenylene of the formula (I)

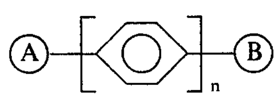
(I)

wherein n is 1 or 2 and the rings A and B, which are identical or different, are trans-4-alkylcyclohexyl or 4-alkylcyclohex-1-enyl, the alkyl groups in each case containing up to 10 carbon atoms.

8. Electro-optical display element based on an liquid crystal cell, characterised in that it contains a liquid crystal dielectric according to Claim 7.

**Claims for the contracting state: AT**

1. Use of the partially hydrogenated oligo-1,4-phenylenes of the formula (I)

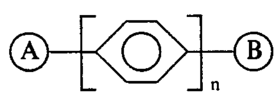
(I)

wherein n is 1 or 2 and the rings A and B, which are identical or different, are trans-4-alkylcyclohexyl or 4-alkylcyclohex-1-enyl, the alkyl groups in each case containing up to 10 carbon atoms, as components of liquid crystal dielectrics.

2. Liquid crystal dielectric for electro-optical display elements, characterised in that it contains at least one partially hydrogenated oligo-1,4-phenylene of the formula (I)

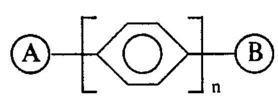
(I)

wherein n is 1 or 2 and the rings A and B, which are identical or different, are trans-4-alkylcyclohexyl or 4-alkylcyclohex-1-enyl, the alkyl groups in each case containing up to 10 carbon atoms.

3. Electro-optical display element based on a liquid crystal cell, characterised in that it contains a liquid crystal dielectric according to Claim 7.

**Revendications pour les Etats contractants: CH, DE, FR, GB, IT, LI, NL**

1. Oligo-1,4-phénylènes partiellement hydrogénés de formule I

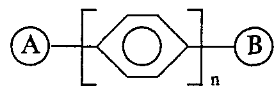
(I)

dans laquelle n est égal à 1 ou 2 et les cycles A et B, identiques ou différents, sont des cycles trans-4-alkylcyclohexyle ou 4-alkylcyclohexène-1-yle, les groupes alkyle contenant chacun jusqu'à 10 atomes de carbone.

2. Oligo-1,4-phénylènes partiellement hydrogénés selon la revendication 1, de formule Ia

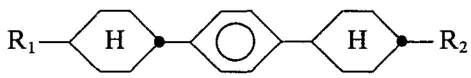
(Ia)

dans laquelle $R_1$ et $R_2$, identiques ou différents, représentent des groupes alkyle contenant jusqu'à 10 atomes de carbone.

3. Oligo-1,4-phénylènes partiellement hydrogénes selon la revendication 1, de formule Ib

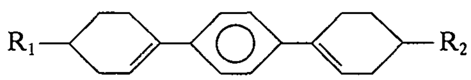
(Ib)

dans laquelle $R_1$ et $R_2$, identiques ou différents, représentent des groupes alkyle contenant jusqu'à 10 atomes de carbone.

4. Oligo-1,4-phénylènes partiellement hydrogénés selon la revendication 1, de formule Ic

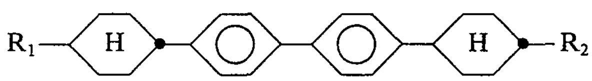
(Ic)

dans laquelle $R_1$ et $R_2$, identiques ou différents, représentent des groupes alkyle contenant jusqu'à 10 atomes de carbone.

5. Oligo-1,4-phénylénes partiellement hydrogénés selon la revendication 1, de formule Id

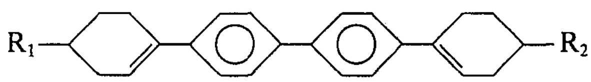
(Id)

dans laquelle $R_1$ et $R_2$, identiques ou différents, représentent des groupes alkyle contenant jusqu'à 10 atomes de carbone.

6. Utilisation des oligo-1,4-phénylénes partiellement hydrogénés de formule I

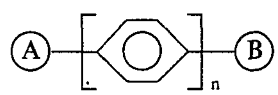
(I)

dans laquelle n est égal à 1 ou 2 et les cycles A et B, identiques ou différents, sont des cycles trans-4-alkylcyclohexyle ou 4-alkylcyclohexène-1-yle, les groupes alkyle contenant chacun jusqu'à 10 atomes de carbone, en tant que composants de diélectriques liquides cristallins.

7. Diélectrique liquide cristallin pour éléments d'affichage électro-optiques caractérisé en ce qu'il contient au moins un oligo-1,4-phénylène partiellement hydrogéné, de formule I

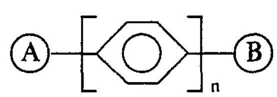
(I)

dans laquelle n est égal à 1 ou 2 et les cycles A et B, identiques ou différents, sont des cycles trans-4-alkylcyclohexyle ou 4-alkylcyclohexène-1-yle, les groupes alkyle contenant chacun jusqu'à 10 atomes de carbone.

8. Elément d'affichage électro-optique à base d'une cellule à cristaux liquides, caractérisé en ce qu'il contient un diélectrique liquide cristallin selon la revendication 7.

**Revendications pour l'etat contractant: AT**

1. Utilisation d'oligo-1,4-phénylènes partiellement hydrogénés de formule I

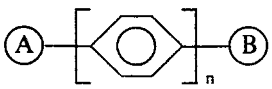

(I)

dans laquelle n est égal à 1 ou 2 et les cycles A et B, identiques ou différents, sont des cycles trans-4-alkylcyclohexyle ou 4-alkylcyclohexène-1-yle, les groupes alkyle contenant chacun jusqu'à 10 atomes de carbone, en tant que composants de diélectriques liquides cristallins.

2. Diélectrique liquide cristallin pour éléments d'affichage électro-optiques, caractérisé en ce qu'il contient au moins un oligo-1,4-phénylène partiellement hydrogéné de formule I

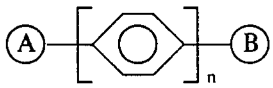

(I)

dans laquelle n est égal à 1 ou 2 et les cycles A et B, identiques ou différents, sont des cycles trans-4-alkylcyclohexyle ou 4-alkylcyclohexène-1-yle, les groupes alkyle contenant chacun jusqu'à 10 atomes de carbone.

3. Elément d'affichage électro-optique à base d'une cellule à cristaux liquides, caractérisé en ce qu'il contient un diélectrique liquide cristallin selon la revendication 2.